# EUROPEAN PATENT SPECIFICATION

(11) **EP 3 835 411 B1**
(45) Date of publication and mention of the grant of the patent: **07.06.2023**
(21) Application number: 20783979.6
(22) Date of filing: 23.03.2020
(51) Int. Cl.: C12N 1/19, C12P 7/22, C12N 15/81, C12N 15/60, C12N 15/53, C12N 15/61, C12R 1/865, C12R 1/645, C12R 1/72, C12R 1/725, C12R 1/65

(54) **RECOMBINANT YEAST, CONSTRUCTION METHOD AND APPLICATION THEREOF IN PREPARING TYROSOL AND DERIVATIVES**
REKOMBINANTE HEFE, VERFAHREN ZU IHRER HERSTELLUNG UND IHRE VERWENDUNG ZUR HERSTELLUNG VON TYROSOL UND DERIVATEN
LEVURE RECOMBINÉE, PROCÉDÉ DE CONSTRUCTION ET APPLICATION CORRESPONDANTE POUR LA PRÉPARATION DE TYROSOL ET DE SES DÉRIVÉS

(30) Priority: 02.04.2019 CN 201910262724; 29.10.2019 WO PCT/CN2019/113879; 07.01.2020 CN 202010012886
(43) Date of publication of application: 16.06.2021
(73) Proprietor: Shandong Henglu biotech.Co., Ltd., Jinan, Shandong (CN)
(72) Inventor: FANG, Xu, Yantai Shandong 264006 (CN); GUO, Wei, Yantai Shandong 264006 (CN); HOU, Shaoli, Yantai Shandong 264006 (CN)
(74) Representative: Sun, Yiming
(86) International application number: PCT/CN2020/080627
(87) International publication number: WO 2020/199952

(56) References cited:
- WO-A1-2014/153036
- CN-A- 108 753 636
- CN-A- 109 929 883
- JP-A- 2018 166 473
- US-A1- 2014 273 165
- US-B2- 10 006 033
- BERGMAN ALEXANDRA ET AL: "Functional expression and evaluation of heterologous phosphoketolases in Saccharomyces cerevisiae", AMB EXPRESS, vol. 6, no. 1, 1 December 2016 (2016-12-01), page 115, XP055847169, DOI: 10.1186/s13568-016-0290-0 Retrieved from the Internet: URL:https://amb-express.springeropen.com/t rack/pdf/10.1186/s13568-016-0290-0.pdf>
- GUO WEI ET AL: "Rational Engineering of Chorismate-Related Pathways in Saccharomyces cerevisiae for Improving Tyrosol Production", FRONTIERS IN BIOENGINEERING AND BIOTECHNOLOGY, vol. 7, 3 July 2019 (2019-07-03), XP055846231, DOI: 10.3389/fbioe.2019.00152
- S. SENTHESHANMUGANATHAN ET AL: "The mechanism of the formation of tyrosol by Saccharomyces cerevisiae", BIOCHEMICAL JOURNAL, vol. 69, no. 2, 1 June 1958 (1958-06-01), pages 210-218, XP055739603, ISSN: 0306-3283, DOI: 10.1042/bj0690210
- GUO WEI ET AL: "Rewiring central carbon metabolism for tyrosol and salidroside production in Saccharomyces cerevisiae", BIOTECHNOLOGY AND BIOENGINEERING, vol. 117, no. 8, 15 May 2020 (2020-05-15), pages 2410-2419, XP055850110, US ISSN: 0006-3592, DOI: 10.1002/bit.27370 Retrieved from the Internet: URL:https://onlinelibrary.wiley.com/doi/fu ll-xml/10.1002/bit.27370>
- Jingjie Jiang et al.: "Metabolic Engineering of Saccharomyces cerevisiae for High-Level Production of Salidroside from Glucose", J Agric Food Chem, vol. 66, no. 17, 19 April 2018 (2018-04-19), XP055587104, ISSN: 0021-8561, DOI: 20200522160918A

## Description

### FIELD OF THE INVENTION

The present invention relates to construction of a recombinant yeast for preparation of tyrosol and derivatives and its application, in particular to a yeast introduced with an expressed gene of exogenous Fructose-6-phosphate phosphoketolase (fxp), and a method for efficiently producing tyrosol and hydroxytyrosol by using the strain, belonging to the technical field of microbial genetic engineering.

### BACKGROUND OF THE INVENTION

Tyrosol is a natural antioxidant derived from olive oil, and is a derivative of phenethyl alcohol. The tyrosol as the aglycone substrate of salidroside is a main medicinal active ingredient of Rhodiola plants and a precursor substance of salidroside and hydroxytyrosol. The tyrosol can protect cells from oxidative damage and is a phenolic compound with important industrial value, the tyrosol and its derivatives are synthetic precursors of a variety of organic compounds, and the tyrosol can be used in a pharmaceutical agent. The hydroxytyrosol as a derivative of the tyrosol has a strong antioxidant effect and multiple physiological and medical functions, and the oxidation resistance of the hydroxytyrosol is stronger than that of the tyrosol. The hydroxytyrosol has been widely used in the industries of biomedicine, functional food, etc., and can prevent the occurrence of cardiovascular diseases, osteopenia, etc. At present, the hydroxytyrosol is mainly extracted from olive leaves. Extraction of hydroxytyrosol from plants is expensive and a lot of arable land was occupied.

Synthesis methods using phenethyl alcohol among the chemical methods mostly protect hydroxyl first, and then obtain p-hydroxyphenylethyl alcohol by means of nitration, reduction, diazotization, and hydrolysis, with a yield of 70%. The phenethyl alcohol is expensive and in short supply. Synthesizing the phenethyl alcohol with nitrotoluene is characterized by low cost, but complex steps and low yield. The phenethyl alcohol is synthesized from p-hydroxystyrene, the yield reaches 96%, and the purity is 99%, but the costs of p-hydroxystyrene are relatively high. The preparation of tyrosol by chemical methods is expensive owe to raw material costs and environment-unfriendliness. These problems directly restrict the industrial production of tyrosol with chemical methods. Therefore, biosynthesis of tyrosol and its derivatives has become a research hotspot.

Tyrosol has the following characteristics: chemical name 4-(2-Hydroxyethyl)phenol, molecular formula C₈H₁₀O₂, molecular weight 138.164, CAS number 501-94-0, and structural formula

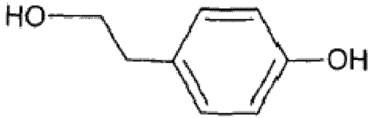

Chinese patent document CN108753636A (application number 201810601213.8) discloses a yeast for producing tyrosol and hydroxytyrosol and a construction method of a recombinant yeast, wherein PcAAS and ADH sequences were introduced into a yeast BY4741 to obtain a PcAAS-ADH recombinant yeast for producing tyrosol; a pdc1 gene knockout cassette and a tyrA expression cassette were introduced into the PcAAS-ADH recombinant yeast to obtain a PcAAS-ADH-Δpdc1-tyrA recombinant yeast for producing tyrosol; and a DNA sequence of HpaBC was introduced into the PcAAS-ADH-Δpdc1-tyrA recombinant yeast to obtain a PcAAS-ADH-HpaBC-Δpdc1-tyrA recombinant yeast for producing hydroxytyrosol. A biosynthesis pathway of tyrosol or hydroxytyrosol was constructed in the BY4741 to increase the output of the tyrosol or hydroxytyrosol. Although this technology can increase the output of tyrosol in the yeast, the output of tyrosol still cannot meet the requirements of industrial production.

Jiang et al., J Agric Food Chem, 2018, vol. 66, no. 17, relates to engineering the production of salidroside in yeast. First, the aromatic aldehyde synthase (AAS) from *Petroselinum crispum* was overexpressed in *Saccharomyces cerevisiae* when combined with endogenous Ehrlich pathway to produce tyrosol from tyrosine.

Glucosyltransferases from different resources were tested for ideal production of salidroside in the yeast. Metabolic flux was enhanced toward tyrosine biosynthesis by overexpressing pathway genes and eliminating feedback inhibition. The pathway genes were integrated into yeast chromosome, leading to a recombinant strain that produced 239.5 mg/L salidroside and 965.4 mg/L tyrosol. The production of salidroside and tyrosol reached up to 732.5 and 1394.6 mg/L, respectively, by fed-batch fermentation.

Bergman et al., AMB EXPRESS, 2016, vol.6, no.1, page 115 discloses a comparison and identification of efficient heterologous phosphoketolase enzyme candidates that in yeast have the potential to reduce carbon loss compared to the native acetyl-CoA producing pathway by redirecting carbon flux directly from C5 and C6 sugars towards C2-synthesis. Nine phosphoketolase candidates were expressed in *S*. *cerevisiae* of which seven produced significant amounts of acetyl-phosphate after provision of sugar phosphate substrates in vitro. The candidates showed differing substrate specificities, and some demonstrated activity levels significantly exceeding those of candidates previously expressed in yeast.

The synthesis of tyrosol in the yeast is affected by a variety of metabolic pathways, and relevant metabolic pathways have not been fully studied. Therefore, how to prepare the tyrosol with fermentation is still a technical problem at present.

### SUMMARY OF THE INVENTION

Aiming at the deficiencies of the prior method, the present invention provides novel uses of recombinant yeasts for preparation of tyrosol and hydroxytyrosol as defined in appended claims.

### BENEFICIAL EFFECTS OF THE PRESENT INVENTION:

1. The present invention discloses for the first time that in the expression process of a yeast, Fructose-6-phosphate is synthesized into beta-D-Fructose 1,6-bisphosphate and also catalyzed into Erythrose-4-phosphate and Acetyl-phosphate, and Xylulose-5-phosphate is catalyzed into Glyceraldehydes-3-phosphate and Acetyl-phosphate, which change the metabolic flux distribution of carbon in the yeast, enhance the synthesis of Erythrose-4-phosphate as an important intermediate for the biosynthesis of tyrosol, optimize the metabolic pathway for synthesizing tyrosol, and increase the yields of tyrosol and its derivatives such as hydroxytyrosol;
2. The expressed gene of exogenous Fructose-6-phosphate phosphoketolase is introduced into the modified yeast cell to obtain a recombinant yeast, which can increase the yield of tyrosol; and the tyrosol is catalyzed by *E. coli* of overexpressed 4-hydroxyphenylacetate hydroxylase to obtain hydroxytyrosol.
3. The present invention provides a novel and environment-friendly biological preparation technology for tyrosol and hydroxytyrosol, which lays a foundation for the large-scale industrial production of tyrosol and hydroxytyrosol, and has important economic value and social benefits.

### BRIEF DESCRIPTION OF THE DRAWING

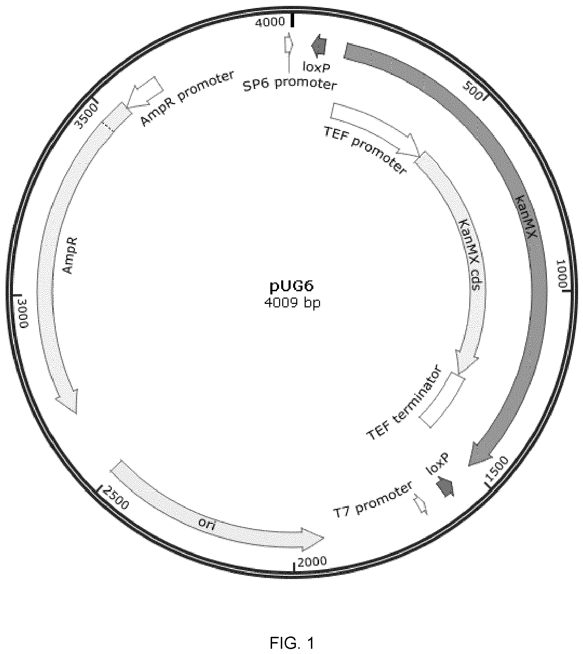

FIG. 1 is a structural diagram of a recombinant plasmid pUG6 in Embodiment 1.

### DETAILED DESCRIPTION OF EMBODIMENTS

It should be noted that the following detailed descriptions are exemplary and are intended to provide further descriptions of the present application. All technical and scientific terms used herein have the same meanings as commonly understood by those ordinary skilled in the art to which the present application belongs, unless specified otherwise.

It should be noted that terms used herein are intended to describe specific implementation modes only rather than to limit the exemplary implementation modes according to the present application. As used herein, the singular form is also intended to comprise the plural form unless otherwise indicated in the context. In addition, it should be understood that when the terms "contain" and/or "comprise" are used in the description, they are intended to indicate the presence of features, steps, operations, devices, components and/or combinations thereof.

The present invention will be further illustrated below in conjunction with embodiments:
In the following embodiments, *E. coli* BL21 and expression vector pET-28a are commercially available. The experimental methods unmarked with specific conditions in the following embodiments were carried out according to conventional conditions, for example, the conditions described in *Molecular Cloning: A Laboratory Manual,* or according to the conditions recommended by the manufacturers of corresponding biological reagents. The PCR amplification reaction process may be a conventional PCR amplification reaction process.

*Saccharomyces cerevisiae* CICC1964, purchased from China Center of Industrial Culture Collection, with a strain number CICC1964, is a known non-collected strain;

According to the method of the patent CN108753636A, the aromatic aldehyde synthase (AAS, EC4.1.1.25) derived from *Petroselinum crispum* was integrated into a delta12 site of *Saccharomyces cerevisiae* CICC1964, and a fused chorismate mutase T /prephenate dehydrogenase (TyrA, EC1.3.1.12, EC 5.4.99.5) derived from *E*. *coli* was substituted for a pdc1 gene of the *Saccharomyces cerevisiae* CICC1964 to obtain an SC-1 strain.

### Embodiment 1

### Construction of Bafxpk and Bbfxpk expression cassettes

Codon optimization was performed on amino acid sequences of SEQ ID No. 1 and SEQ ID No. 2 according to the codon preference of a host *Saccharomyces cerevisiae* to obtain optimized nucleotide sequences SEQ ID No. 3 and SEQ ID No. 4 corresponding to SEQ ID No. 1 and SEQ ID No. 2 for gene synthesis. An expressed gene of a target gene Fructose-6-phosphate phosphoketolase was obtained by amplification with primer pairs Bafxpk-F/Bafxpk-R and Bbfxpk-F/Bbfxpk-R via a Phanta Max High-Fidelity DNA polymerase from Vazyme (the nucleotide sequence of the *Bafxpk* fragment was shown as SEQ ID No. 3, and the nucleotide sequence of the *Bbfxpk* fragment was shown as SEQ ID No. 4). By using a genome of *Saccharomyces cerevisiae* CICC1964 as a template, DNA fragments of forward and reverse homologous arms were amplified with primer pairs U-F/ U-R and D-F/ D-R via PCR, and fragments of a promoter *tpi1* and a terminator *gpm1* were amplified with primer pairs Ptpi1-F/ Ptpi1-R and Tgpm1-F/ Tgpm1-R. A DNA fragment of a resistance gene KanMX4 was amplified with primer pairs G418-F/ G418-R via PCR, using DNA with a recombinant plasmid pUG6 (as shown in FIG. 1) of a geneticin resistance gene KanMX4 (the nucleotide sequence was shown as SEQ ID No. 24) as a template. After the size of a band was verified by agarose gel electrophoresis to be correct, the band was cut, and gene fragments were recovered with an OMEGA gel extraction kit. The primers for PCR amplification were as follows:
The sequences of the primer pair Bafxpk-F/Bafxpk-R were SEQ ID No. 13 and SEQ ID No. 14; the sequences of the primer pair Bbfxpk-F/Bbfxpk-R were SEQ ID No. 15 and SEQ ID No. 16; the sequences of the primer pair U-F/ U-R were SEQ ID No. 5 and SEQ ID No. 6; the sequences of the primer pair D-F/ D-R were SEQ ID No. 7 and SEQ ID No. 8; the sequences of the primer pair Ptpi1-F/ Ptpi1-R were SEQ ID No. 9 and SEQ ID No. 10; the sequences of the primer pair Tgpm1-F/ Tgpm1-R were SEQ ID No. 11 and SEQ ID No. 12; and the sequences of the primer pair G418-F/ G418-R were SEQ ID No. 17 and SEQ ID No. 18;

The target gene fragments were amplified with Phanta Max High-Fidelity DNA polymerase, each fragment was ensured to have a 50 bp homologous sequence with an adjacent fragment, a PCR product was recovered with a DNA fragment gel recovery kit after the gel electrophoresis, and DNA concentration was measured. Then, the purified DNA fragments with homologous sequences were fused by a fusion PCR method:
(1) The fragments were ligated by using Phanta Max High-Fidelity DNA polymerase. The reaction system was shown as Table 2:

**Table 2 Reaction system**

| Composition | Adding amount (µL) |
|---|---|
| DNA fragments | 200 ng each |
| 2 x Phanta Max Buffer 2 | 2 |
| dNTP Mix (10 Mm each) | 0.2 |
| Phanta Max High-Fidelity DNA polymerase | 0.5 |
| dd H₂O | Make up to 20 µL |
| Total | 25 |

The above reagents were added to a PCR tube, and the reaction conditions were shown as Table 3: 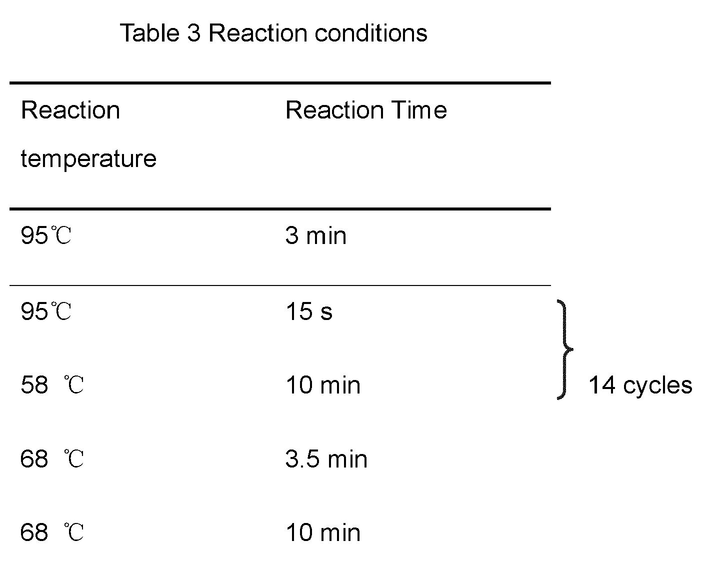 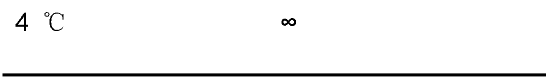
(2) By using the PCR product of (1) as a PCR amplification template, the obtained target fragments were amplified with a primer pair Yzaw-F/ Yzaw-R via Phanta Max High-Fidelity DNA from Vazyme. After the size of the band was verified by agarose gel electrophoresis to be correct, the band was cut, and DNA fragments, i.e., DNA fragments of *Bafxpk* and *Bbfxpk* expression cassettes, were recovered with the OMEGA gel extraction kit. The primers for PCR amplification were as follows:
The sequence of Yzaw-F was SEQ ID No. 19; the sequence of Yzaw-R was SEQ ID No. 20;
(3) The DNA fragments of the *Bafxpk* and *Bbfxpk* expression cassettes obtained in (2) were verified by sequencing.

### Embodiment 2

### Construction of Bafxpk and Bbfxpk heterologous expression strains, taking Saccharomyces cerevisiae as an example:

*Saccharomyces cerevisiae* tyrosol synthesis strain CICC1964 was transformed by a PEG/LiAc method, resistance G418 was added to a medium for screening the strain, a genome was extracted, PCR verification was performed by using a primer pair Yzaw-F/ Yzaw-R, and SC-bafxpk and SC-bbfxpk strains were obtained.

### Embodiment 3

### Fermentation of microorganisms for synthesizing tyrosol, taking Saccharomyces cerevisiae as an example:

The yeasts were picked from a plate of strains CICC1964, SC-bafxpk and SC-bbfxpk for producing tyrosol, inoculated to a 5 mL YPD liquid medium, cultured under the conditions of 30-32°C and 200 rpm for 24 h, and transferred to a 50 mL YPD liquid medium, wherein the initial inoculation OD₆₀₀ was 0.2; the broths were cultured under the conditions of 30°C and 200 rpm for 12 h, and then transferred to a 100 mL YPD liquid medium, wherein the initial inoculation OD₆₀₀ was 0.2, and the medium contained a carbon source such as 2% glucose or 2% sucrose or 2% glucose and 1% tyrosine; and after 24 hours of culture, the carbon source such as 2% glucose or 2% sucrose or 2% glucose and 1% tyrosine was added again for a total of 72 hours of fermentation. The concentration of tyrosol in the fermentation broth was tested by the HPLC method reported in the literature (Satoh et al., Journal of Agricultural and Food Chemistry, 60, 979-984, 2012). The yields of tyrosol under different carbon source culture conditions were shown in Table 4.

**Table 4 Yields of tyrosol after 72 hours of fermentation under different carbon sources**

| **Strain (mg/L)** | **Glucose (mg/L)** | **Sucrose (mg/L)** | **Glucose + tyrosine (mg/L)** |
|---|---|---|---|
| SC-bafxp | 3103.16 | 3240.37 | 3526.18 |
| SC-bbfxpk | 3218.41 | 3400.23 | 3684.27 |
| SC-1 | 890.38 | 906.14 | 113.75 |

### Embodiment 4

### Method for obtaining gene HpaBC and E. coli expression vector

A DNA sequence gene cluster of 4-hydroxyphenylacetate 3-hydroxylase (HpaBC, enzyme system number EC 1.5.1.37) derived from E. *coli* was overexpressed in E. *coli,* and the tyrosol was catalyzed into hydroxytyrosol by using whole E. *coli* cells.

An amino acid sequence gene cluster of the 4-hydroxyphenylacetate 3-hydroxylase included: the amino acid sequence of 4-hydroxyphenylacetate hydroxylase (HpaB) was SEQ ID No. 21, and the corresponding nucleotide sequence was SEQ ID NO. 23; the amino acid sequence of 4-hydroxyphenylacetate hydroxylase (HpaC) was SEQ ID No. 22, and the corresponding nucleotide sequence was SEQ ID NO. 24.

An *E*. *coli* DE3 genome was extracted as a template by using a bacterial genome kit, the SEQ ID No. 23 and the SEQ ID No. 24 were amplified with primer pairs hpaB-F/hpaB-R and hpaC-F/hpaC-R, respectively, and sequencing verification was performed. The sequence of hpaB-F was SEQ ID No. 25; the sequence of hpaB-R was SEQ ID No. 26; the sequence of hpaC-F was SEQ ID No. 27; and the sequence of hpaC-R was SEQ ID No. 28.

*E. coli* containing a pET-28a empty vector was cultured with pET-28a as an expression vector, pET-28a plasmids were extracted by using a bacterial plasmid extraction kit, and an expression vector pEThpaBC (SEQ ID NO. 29) was constructed by using a conventional molecular biology method. Then, the pEThpaBC was transformed into an *E*. *coli* expression vector BL21, and kanamycin was used as a selection marker to obtain monoclonal BL21-pEThpaBC.

The BL21-pEThpaBC was cultured by shaking, and the expression of HPAB/C was induced by using 1 mM IPTG.

The obtained BL21-pEThpaBC strain was added to the medium of Embodiment 3. After reacting for 3 hours, the yields of hydroxytyrosol obtained were tested. The results were shown in Table 5:

**Table 5 Yields of hydroxytyrosol after adding BL21-pEThpaBC strain to the medium shown in Table 4 and mixing for 3 hours**

| **Strain (mg/L)** | **Glucose (mg/L)** | **Sucrose (mg/L)** | **Glucose + tyrosine (mg/L)** |
|---|---|---|---|
| SC-bafxp | 3010.34 | 2989.15 | 3268.39 |
| SC-bbfxpk | 3156.81 | 3008.51 | 3409.83 |
| SC-1 | 840.28 | 850.31 | 101.25 |

### Embodiment 5

*Kluyveromyces marxianus* with a strain number NBRC1777 was used. According to the method of the patent CN108753636A, an aromatic aldehyde synthase (AAS, EC4.1.1.25) derived from *Petroselinum crispum* was integrated into a delta12 site of the *Kluyveromyces marxianus,* and a fused chorismate mutase T /prephenate dehydrogenase (TyrA, EC1.3.1.12, EC 5.4.99.5) derived from *E*. *coli* was substituted for a pdc1 gene of the *Kluyveromyces marxianus* to obtain a *Kluyveromyces marxianus* strain with a metabolic pathway for synthesizing tyrosol via Erythrose-4-phosphate and phosphoenolpyruvate.

KM-bafxp and KM-bbfxp strains were prepared via Fructose-6-phosphate phosphoketolase (EC 4.1.2.22) according to the methods described in Embodiments 1-2 of the present invention, and the difference was that the culture temperature of *Kluyveromyces marxianus* cells was 42-49°C.

### Embodiment 6

Fructose-6-phosphate phosphoketolase (bdfxp, GenBank: BAQ26957.1) derived from *Bifidobacterium dentium* was used, with an amino acid sequence shown as SEQ ID No. 30. Tyrosol and hydroxytyrosol were prepared according to the methods described in Embodiment 3 and Embodiment 4. The sequences of a primer pair Bdfxp-F/Bdfxp-R used were SEQ ID No. 32 and SEQ ID No. 33. The results were similar to those of Embodiment 3 and Embodiment 4, i.e., the yields of tyrosol and hydroxytyrosol were also increased. The specific results were as follows:

**Table 6 Yields of tyrosol after 72 hours of fermentation under different carbon sources**

| **Strain (mg/L)** | **Glucose (mg/L)** | **Sucrose (mg/L)** | **Glucose + tyrosine (mg/L)** |
|---|---|---|---|
| SC-bdfxp | 3194.84 | 3215.46 | 3570.07 |

**Table 7 Yields of hydroxytyrosol after adding BL21-pEThpaBC strain to the medium shown in Table 6 and mixing for 3 hours**

| **Strain (mg/L)** | **Glucose (mg/L)** | **Sucrose (mg/L)** | **Glucose + tyrosine (mg/L)** |
|---|---|---|---|
| SC-bdfxp | 3209.89 | 3146.29 | 3310.81 |

### Embodiment 7

According to the methods described in Embodiments 1-2, an aromatic aldehyde synthase (AAS, EC4.1.1.25) derived from *Petroselinum crispum* was integrated into a delta12 site of *Saccharomyces cerevisiae* CICC1964, and Fructose-6-phosphate phosphoketolase (EC 4.1.2.22) as shown in SEQ ID NO. 4 was introduced into the yeast to obtain an SC-bbfxpk-AAS strain. The result was similar to the tyrosol yield of the SC-bbfxpk strain in Embodiment 3.

The obtained BL21-pEThpaBC strain was added to the prepared medium containing tyrosol. After reacting for 3 hours, the yield of hydroxytyrosol obtained was tested, and the result was also similar to that of the SC-bbfxpk strain described in Embodiment 4. It showed that similar yields of tyrosol and hydroxytyrosol can also be obtained without introducing a fused chorismate mutase T /prephenate dehydrogenase (TyrA, EC1.3.1.12, EC 5.4.99.5) gene into the yeast. The specific results were shown in Table 8 and Table 9:

**Table 8 Yields of tyrosol after 72 hours of fermentation under different carbon sources**

| **Strain (mg/L)** | **Glucose (mg/L)** | **Sucrose (mg/L)** | **Glucose + tyrosine (mg/L)** |
|---|---|---|---|
| SC-bbfxpk-A | 3291.76 | 3402.13 | 3702.09 |
| AS | | | |
| SC-bbfxpk | 3218.41 | 3400.23 | 3684.27 |

**Table 9 Yields of hydroxytyrosol after adding BL21-pEThpaBC strain to the medium shown in Table 8 and mixing for 3 hours**

| **Strain (mg/L)** | **Glucose (mg/L)** | **Sucrose (mg/L)** | **Glucose + tyrosine (mg/L)** |
|---|---|---|---|
| SC-bbfxpk-A AS | 3202.65 | 3015.77 | 3432.72 |
| SC-bbfxpk | 3156.81 | 3008.51 | 3409.83 |

### Embodiment 8

According to the method described in Embodiment 6, gene fragments corresponding to Fructose-6-phosphate phosphoketolase (baifxp, GenBank: WP_052826255.1) with an amino acid sequence SEQ ID No. 100 derived from *Bifidobacterium animalis,* Fructose-6-phosphate phosphoketolase (bbifxp, GenBank: WP_047289945.1) with an amino acid sequence SEQ ID No. 101 derived from *Bifidobacterium bifidum,* Fructose-6-phosphate phosphoketolase (blafxp, GenBank: CAC29121.1) with an amino acid sequence SEQ ID No. 102 derived from *Bifidobacterium lactis,* Fructose-6-phosphate phosphoketolase (blofxp, GenBank: PWH09343.1) with an amino acid sequence SEQ ID No. 103 derived from *Bifidobacterium longum,* Fructose-6-phosphate phosphoketolase (bmfxp, GenBank: CAC29121.1) with an amino acid sequence SEQ ID No. 104 derived from *Bifidobacterium mongoliense,* Fructose-6-phosphate phosphoketolase (bpfxp, GenBank: WP_034883174.1) with an amino acid sequence SEQ ID No. 105 derived from *Bifidobacterium pseudolongum,* Fructose-6-phosphate phosphoketolase (Anfxp, GenBank: CBF76492.1) with an amino acid sequence SEQ ID No. 106 derived from *Aspergillus nidulans,* Fructose-6-phosphate phosphoketolase (Cafxp, GenBank: KHD36088.1) with an amino acid sequence SEQ ID No. 107 derived from *Clostridium acetobutylicum,* Fructose-6-phosphate phosphoketolase (Lmfxp, GenBank: AAV66077.1) with an amino acid sequence SEQ ID No. 108 derived from *Leuconostoc mesenteroides,* Fructose-6-phosphate phosphoketolase (Lprfxp, GenBank: ALO04878.1) with an amino acid sequence SEQ ID No. 109 derived from *Lactobacillus paraplantarum,* and Fructose-6-phosphate phosphoketolase (Lplfxp, GenBank: KRU19755.1) with an amino acid sequence SEQ ID No. 110 derived from *Lactobacillus plantarum,* were transformed into modified *Saccharomyces cerevisiae;* modified strains SC-baifxp, SC-bbifxp, SC-blafxp, SC-blofxp, SC-bmfxp, SC- bpfxp, SC-Anfxp, SC-Cafxp, SC-Lmfxp, SC-Lprfxp, and SC-Lplfxp were obtained, respectively; and the modified *Saccharomyces cerevisiae* was obtained by integrating an aromatic aldehyde synthase derived from *Petroselinum crispum* into a delta12 of *Saccharomyces cerevisiae* CICC1964. The results were similar to those of Embodiment 3 and Embodiment 4, i.e., the yields of tyrosol and hydroxytyrosol were also increased. The specific results were shown in Table 10 and Table 11.

**Table 8 Yields of tyrosol after 72 hours of fermentation under different carbon sources**

| **Strain (mg/L)** | **Glucose (mg/L)** | **Sucrose (mg/L)** | **Glucose + tyrosine (mg/L)** |
|---|---|---|---|
| SC-baifxp | 1484.214 | 1425.86 | 1625.11 |
| SC-bbifxp | 1522.71 | 1563.47 | 1699.25 |
| SC-blafxp | 1658.23 | 1681.93 | 1821.61 |
| SC-blofxp | 1828.46 | 1900.84 | 2134.61 |
| SC-bmfxp | 1400.19 | 1456.91 | 1611.88 |
| SC-bpfxp | 1233.41 | 1290.41 | 1390.42 |
| SC-Anfxp | 927.04 | 940.71 | 1004.84 |
| SC-Cafxp | 1604.22 | 1612.88 | 1824.61 |
| SC-Lmfxp | 956.17 | 943.45 | 945.21 |
| SC-Lprfxp | 1200.02 | 1210.32 | 1423.56 |
| SC-Lplfxp | 1128.23 | 1194.16 | 1256.23 |

**Table 9 Yields of hydroxytyrosol after adding BL21-pEThpaBC strain to the medium shown in Table 8 and mixing for 3 hours**

| **Strain (mg/L)** | **Glucose (mg/L)** | **Sucrose (mg/L)** | **Glucose + tyrosine (mg/L)** |
|---|---|---|---|
| SC-baifxp | 1249.89 | 1246.78 | 1319.71 |
| SC-bbifxp | 1386.09 | 1364.21 | 1402.34 |
| SC-blafxp | 1423.02 | 1401.32 | 1634.09 |
| SC-blofxp | 1614.02 | 1715.56 | 1973.21 |
| SC-bmfxp | 1267.01 | 1301.11 | 1472.01 |
| SC-bpfxp | 1011.33 | 1199.21 | 1187.43 |
| SC-Anfxp | 871.21 | 877.09 | 903.89 |
| SC-Cafxp | 1500.09 | 1490.36 | 1614.70 |
| SC-Lmfxp | 799.23 | 789.02 | 801.90 |
| SC-Lprfxp | 1000.82 | 1060.11 | 1225.43 |
| SC-Lplfxp | 997.13 | 909.61 | 1023.31 |

From the above results, when the above exogenous Fructose-6-phosphate phosphoketolases were expressed in yeast cells, the yields were different and lower than those using the Fructose-6-phosphate phosphoketolases derived from *Bifidobacterium adolescentis, Bifidobacterium breve* and *Bifidobacterium dentium* in Embodiment 3, Embodiment 4 and Embodiment 5, but compared with original strain, the yields of tyrosol and hydroxytyrosol were significantly increased.

Based on the results of Embodiment 7, the inventors simultaneously transformed gene fragments corresponding to the above Fructose-6-phosphate phosphoketolases into SC-1 strain obtained according to the method of the invention patent application (application number 201810601213.8), i.e.: an aromatic aldehyde synthase (AAS, EC4.1.1.25) derived from *Petroselinum crispum* was integrated into a delta12 site of *Saccharomyces cerevisiae* CICC1964, and a fused chorismate mutase T /prephenate dehydrogenase (TyrA, EC1.3.1.12, EC 5.4.99.5) derived from *E. coli* is substituted for a pdc1 gene of the *Saccharomyces cerevisiae* CICC1964; the results showed that the results of baifxp, bbifxp, blafxp, blofxp, bmfxp, bpfxp, Anfxp, Cafxp, Lmfxp, Lprfxp, and Lplfxp were similar to the results of bbfxpk in Embodiment 7, indicating whether the fused chorismate mutase T /prephenate dehydrogenase (TyrA, EC1.3.1.12, EC 5.4.99.5) gene was introduced into the yeast, the yields of tyrosol and hydroxytyrosol were affected a little, with universality.

### RESULT ANALYSIS

Based on the above results, it can be found by those skilled in the art that when exogenous Fructose-6-phosphate phosphoketolase was expressed in yeast cells, the yield of tyrosol can be significantly increased, and this phenomenon was not limited to one source of Fructose-6-phosphate phosphoketolase and specific yeast cells; it can be expected by those skilled in the art through the technical teaching of the present invention that, after the Fructose-6-phosphate phosphoketolase was expressed in the yeast cells with metabolic pathways for synthesizing tyrosol via Erythrose-4-phosphate and phosphoenolpyruvate, because the expression of the metabolic exogenous Fructose-6-phosphate phosphoketolase affected the sugar metabolism pathways of the yeast cells, the production of tyrosol products was promoted, and the technical effects described in the present application can be achieved.

## Claims

1. Use of a recombinant yeast in production of tyrosol, wherein the recombinant yeast contains an expressed gene of exogenous Fructose-6-phosphate phosphoketolase derived from *Bifidobacterium adolescentis, Bifidobacterium breve, Bifidobacterium dentium,* or *Bifidobacterium longum.*

2. The use of a recombinant yeast in production of tyrosol according to claim 1, wherein the recombinant yeast cell is obtained by integrating an aromatic aldehyde synthase and the fused chorismate mutase / prephenate dehydrogenase of T-protein into the genome.

3. The use of a recombinant yeast in production of tyrosol according to claim 1 or 2, wherein the yeast cell is: *Saccharomyces cerevisiae, Yarrowia lipolytica, Schizosaccharomyces pombe, Kluyveromyces lactis, Kluyveromyces marxianus, Candida lipolytica, Torulopsis glabrata, Rhodotorula glutinis, Rhodotorula graminis, Saccharomyces pastorianus, Candida tropicalis, Zygosaccharomyces rouxii, Candida glabrata, Torulaspora delbrueckii, Debaryomyces hansenii, Scheffersomyces stipites, Meyerozyma guilliermondii, Lodderomyces elongisporus, Candida albicans, Candida orthopsilosis, Candida metapsilosis, Candida dubliniensis, Clavispora lusitaniae,* or *Candida auris.*

4. The use of a recombinant yeast in production of tyrosol according to claim 1 or 2, wherein the amino acid sequence of the Fructose-6-phosphate phosphoketolase is shown as SEQ ID No. 1 or SEQ ID No. 2, or SEQ ID No. 30.

5. The use of a recombinant yeast in production of tyrosol according to claim 1 or 2, wherein the yeast cell is *Saccharomyces cerevisiae* with a strain number CICC1964 or the *Kluyveromyces marxianus* with a strain number NBRC1777.

6. The use of a recombinant yeast in production of tyrosol according to claim 1 or 2, wherein the modified yeast cell comprises an aromatic aldehyde synthase derived from *Petroselinum crispum* integrated into a delta12 of *Kluyveromyces marxianus* NBRC1777, and the fused chorismate mutase / prephenate dehydrogenase of T-protein derived from *E*. *coli* substituted for a pdc1 gene of *Saccharomyces cerevisiae* CICC1964.

7. The use of the recombinant yeast in production of tyrosol according to claim 1 or 2, wherein the fermentation medium for fermentation contains at least one or a combination of two or more of glucose, fructose and sucrose, and tyrosine.

8. Use of a recombinant yeast in production of hydroxytyrosol, wherein the recombinant yeast containing an expressed gene of exogenous Fructose-6-phosphate phosphoketolase derived from *Bifidobacterium adolescentis, Bifidobacterium breve, Bifidobacterium dentium,* or *Bifidobacterium longum* is used for tyrosol production by fermentation, optionally wherein the recombinant yeast cell is obtained by integrating an aromatic aldehyde synthase and the fused chorismate mutase / prephenate dehydrogenase of T-protein into the genome, and wherein after being prepared through fermentation, tyrosol is reacted with 4-hydroxyphenylacetate hydroxylase to obtain hydroxytyrosol.

9. The use of a recombinant yeast in production of hydroxytyrosol according to claim 8, wherein the fermentation medium for the use contains at least one or a combination of two or more of glucose, fructose and sucrose, and tyrosine.

## Patentansprüche

1. Verwendung einer rekombinanten Hefe bei der Herstellung von Tyrosol, wobei die rekombinante Hefe ein exprimiertes Gen von exogener Fructose-6-phosphat-Phosphoketolase enthält, das aus *Bifidobacterium adolescentis, Bifidobacterium breve, Bifidobacterium dentium* oder *Bifidobacterium longum* stammt.

2. Verwendung einer rekombinanten Hefe bei der Herstellung von Tyrosol nach Anspruch 1, wobei die rekombinante Hefezelle durch Integration einer aromatischen Aldehydsynthase und der fusionierten Chorismatmutase/Prephenatdehydrogenase des T-Proteins in das Genom erhalten wird.

3. Verwendung einer rekombinanten Hefe bei der Produktion von Tyrosol nach Anspruch 1 oder 2, wobei die Hefezelle folgende ist: *Saccharomyces cerevisiae, Yarrowia lipolytica, Schizosaccharomyces pombe, Kluyveromyces lactis, Kluyveromyces marxianus, Candida lipolytica, Torulopsis glabrata, Rhodotorula glutinis, Rhodotorula graminis, Saccharomyces pastorianus, Candida tropicalis, Zygosaccharomyces rouxii, Candida glabrata, Torulaspora delbrueckii, Debaryomyces hansenii, Scheffersomyces stipites, Meyerozyma guilliermondii, Lodderomyces elongisporus, Candida albicans, Candida orthopsilosis, Candida metapsilosis, Candida dubliniensis, Clavispora lusitaniae* oder *Candida a*uris.

4. Verwendung einer rekombinanten Hefe bei der Produktion von Tyrosol nach Anspruch 1 oder 2, wobei die Aminosäuresequenz der Fructose-6-phosphat-Phosphoketolase als SEQ ID Nr. 1 oder SEQ ID Nr. 2 oder SEQ ID Nr. 30 dargestellt wird.

5. Verwendung einer rekombinanten Hefe bei der Produktion von Tyrosol nach Anspruch 1 oder 2, wobei die Hefezelle *Saccharomyces cerevisiae* mit der Stammnummer CICC1964 oder *Kluyveromyces marxianus* mit der Stammnummer NBRC1777 ist.

6. Verwendung einer rekombinanten Hefe bei der Produktion von Tyrosol nach Anspruch 1 oder 2, wobei die modifizierte Hefezelle eine aromatische Aldehydsynthase aus *Petroselinum crispum* umfasst, die in eine delta12 von *Kluyveromyces marxianus* NBRC1777 integriert ist und die fusionierte Chorismatmutase/Prephenatdehydrogenase des T-Proteins aus *E*. *coli* für ein pdc1 Gen von *Saccharomyces cerevisiae* CICC1964 ausgetauscht ist.

7. Verwendung der rekombinanten Hefe zur Herstellung von Tyrosol nach Anspruch 1 oder 2, wobei das Fermentationsmedium für die Fermentation mindestens eines oder eine Kombination von zwei oder mehreren von Glucose, Fructose und Saccharose, und Tyrosin enthält.

8. Verwendung einer rekombinanten Hefe bei der Herstellung von Hydroxytyrosol, wobei die rekombinante Hefe, die ein exprimiertes Gen von exogener Fructose-6-phosphat-Phosphoketolase aus *Bifidobacterium adolescentis, Bifidobacterium breve, Bifidobacterium dentium* oder *Bifidobacterium longum* enthält, zur Tyrosolherstellung durch Fermentation verwendet wird, wobei wahlweise die rekombinante Hefezelle durch Integrieren einer aromatischen Aldehydsynthase und der fusionierten Chorismatmutase/Prephenatdehydrogenase des T-Proteins in das Genom erhalten wird, und wobei Tyrosol, nachdem es durch Fermentation hergestellt wurde, mit 4-Hydroxyphenylacetat-Hydroxylase umgesetzt wird, um Hydroxytyrosol zu erhalten.

9. Verwendung einer rekombinanten Hefe bei der Herstellung von Hydroxytyrosol nach Anspruch 8, wobei das Fermentationsmedium für die Verwendung mindestens eines oder eine Kombination von zwei oder mehreren von Glucose, Fructose und Saccharose, und Tyrosin enthält.

## Revendications

1. Utilisation d'une levure recombinée dans la production de tyrosol, dans laquelle la levure recombinée contient un gène exprimé de la fructose-6-phosphate phosphocétolase dérivée de *Bifidobacterium adolescentis, Bifidobacterium animalis, Bifidobacterium dentium* ou *Bifidobacterium longum.*

2. Utilisation d'une levure recombinée dans la production de tyrosol selon la revendication 1, dans laquelle la cellule de levure recombinée est obtenue par intégration d'une aldéhyde synthase aromatique et de la chorismate mutase/préphénate déshydrogénase fusionnée de la protéine T dans le génome.

3. Utilisation d'une levure recombinée dans la production de tyrosol selon la revendication 1 ou 2, dans laquelle la cellule de levure est : *Saccharomyces cerevisiae, Yarrowia lipolytica, Schizosaccharomyces pombe, Kluyveromyces lactis, Kluyveromyces marxianus, Candida lipolytica, Torulopsis glabrata, Rhodotorula glutinis, Rhodotorula graminis, Saccharomyces pastorianus, Candida tropicalis, Zygosaccharomyces rouxii, Candida glabrata, Torulaspora delbrueckii, Debaryomyces hansenii, Scheffersomyces stipites, Meyerozyma guilliermondii, Lodderomyces elongisporus, Candida albicans, Candida orthopsilosis, Candida metapsilosis, Candida dubliniensis, Clavispora lusitaniae,* ou *Candida auris.*

4. Utilisation d'une levure recombinée dans la production de tyrosol selon la revendication 1 ou 2, dans laquelle la séquence d'acides aminés de la fructose-6-phosphate phosphocétolase est présentée comme SEQ ID NO : 1 ou SEQ ID NO : 2, ou SEQ ID NO : 30.

5. Utilisation d'une levure recombinée dans la production de tyrosol selon la revendication 1 ou 2, dans laquelle la cellule de levure est *Saccharomyces cerevisiae* avec un numéro de souche CICC1964 ou *Kluyveromyces marxianus* avec un numéro de souche NBRC1777.

6. Utilisation d'une levure recombinée dans la production de tyrosol selon la revendication 1 ou 2, dans laquelle la cellule de levure modifiée comprend une aldéhyde synthase aromatique dérivée de *Petroselinum crispum* intégré dans un delta12 de *Kluyveromyces marxianus* NBRC1777, et la chorismate mutase/préphénate déshydrogénase fusionnée de la protéine T dérivée d'E. *coli* substituée à un gène pdc1 de *Saccharomyces cerevisiae* CICC1964.

7. Utilisation d'une levure recombinée dans la production de tyrosol selon la revendication 1 ou 2, dans laquelle le milieu de fermentation pour la fermentation contient au moins un ou une combinaison de deux ou plusieurs du glucose, du fructose et du sucrose, et de la tyrosine.

8. Utilisation d'une levure recombinée dans la production d'hydroxytyrosol, dans laquelle la levure recombinée contenant un gène exprimé de la fructose-6-phosphate phosphocétolase dérivée de *Bifidobacterium adolescentis, Bifidobacterium animalis, Bifidobacterium dentium* ou *Bifidobacterium longum* est utilisée pour la production de tyrosol par fermentation, facultativement dans laquelle la cellule de levure recombinée est obtenue par intégration d'une aldéhyde synthase aromatique et de la chorismate mutase/préphénate déshydrogénase fusionnée de la protéine T dans le génome, et dans lequel après avoir été préparé par fermentation, le tyrosol est mis en réaction avec la 4-hydroxyphénylacétate hydroxylase pour obtenir l'hydroxytyrosol.

9. Utilisation d'une levure recombinée dans la production d'hydroxytyrosol selon la revendication 8, dans laquelle le milieu de fermentation à utiliser contient au moins l'un ou une combinaison de deux ou plus du glucose, du fructose et du sucrose, et de la tyrosine.
